Europäisches Patentamt

European Patent Office

Office européen des brevets

(11) Veröffentlichungsnummer: **0 167 710**
**A1**

## (12) EUROPÄISCHE PATENTANMELDUNG

(21) Anmeldenummer: 85103325.8

(22) Anmeldetag: 21.03.85

(51) Int. Cl.⁴: **A 61 K 31/415**
//C07D235/32

(30) Priorität: 21.03.84 DE 3410348

(43) Veröffentlichungstag der Anmeldung:
15.01.86 Patentblatt 86/3

(84) Benannte Vertragsstaaten:
AT BE CH DE FR GB IT LI LU NL SE

(71) Anmelder: Eduro World Investments Inc.
Comosa Building Samuel Lewis Avenue E.P. 1
Panama(PA)

(72) Erfinder: Carboni, Giovanni
Via 14 IA Pitentino 4a
I-24100 Bergamo(IT)

(72) Erfinder: Schreiber, Christian
Via Cavour
I-24061 Albano S Alessandro(IT)

(74) Vertreter: Huber, Bernhard, Dipl.-Chem. et al,
Patentanwälte H. Weickmann, Dr. K. Fincke F.A.
Weickmann, B. Huber Dr. H. Liska, Dr. J. Prechtel
Möhlstrasse 22 Postfach 860 820
D-8000 München 86(DE)

(54) Verfahren zur Bekämpfung von Fischmykosen und Mittel zur Durchführung des Verfahrens.

(57) Es wird ein Verfahren zur Bekämpfung von Fischmykosen, insbesondere von Ichtiophtyriasis beschrieben, bei dem
man dem Fischwasser als Wirkstoff 1-(n-Butylcarbamoyl)-2-
(methoxy-carbonylamino)-benzimidazol und/oder 2-
(Methoxycarbonylamino)-benzimidazol, vorzugsweise zusammen mit Ligninsulfonaten und gegebenenfalls zusammen mit biologisch aktiven Nährstoffen zusetzt. Ein weiterer
Gegenstand ist ein Mittel zur Durchführung des Verfahrens,
das die Wirkstoffe und ein Ligninsulfonat enthält, und
gegebenenfalls biologisch aktive Nährstoffe, übliche Träger-
und/oder Hilsstoffe.

# Beschreibung

Die Erfindung betrifft ein Arzneimittel, insbesondere
zur Bekämpfung von Erkrankungen der Außenhaut von
Wassertieren.

Erkrankungen der Außenhaut sind eine unerwünschte,
sowohl in Erwerbsfischzuchten wie auch in Aquarien sehr
häufig auftretende Erscheinung. Tierpräsitäre Erreger
und die damit verursachten Krankheiten, insbesondere
Ichthyophthyriasis und andere Protozoenerkrankungen
wurden bisher vorwiegend mit Mitteln bekämpft, die z.
B. Malachitgrün oder Kuperionen als Wirkstoffe enthalten.

Eine solche Behandlung ist aber mit erheblichen Nachteilen verbunden, insbesondere weil z. B. kupferhaltige
Mittel über längere Zeit (bis zu ca. 2 Wochen) in
erheblichen Mengen zugefügt werden müssen. Dabei läßt
es sich nicht ausschließen, daß häufig eine Konzentration erreicht wird, die auf die Fische bereits toxisch
wirkt und den Tod der Tiere zur Folge hat. Dazu kommt
noch, daß selbst bei hohen Konzentrationen, die in der
Nähe der Toxizitätsgrenze liegen oder bei wiederholter
Anwendung oft nur eine mittelmäßige Wirkung und bei
einigen Krankheiten sogar nur ein sehr geringer Erfolg
erzielt wird. Der Zusatz erheblicher Mengen an kupferhaltigen Mitteln ist außerdem auch mit dem Nachteil der
Belastung des Wassers mit Schadstoffen verbunden,
wodurch eine auch zeitlich aufwendige Erneuerung des
Wassers oft schon während, aber zumindest nach der Behandlung notwendig wird.

Malachitgrün erweist sich zwar bei der Behandlung von Fischkrankheiten als sehr wirkungsvoll, hat jedoch teratogene Wirkungen und färbt Wasser und Haut in unerwünschter Weise.

Aufgabe der vorliegenden Erfindung ist es deshalb, die bestehenden Nachteile zu überwinden und ein Verfahren bzw. ein Mittel zur Bekämpfung von Fischkrankheiten, insbesondere von Ichthyophthyriasis bereitzustellen, mit dem bereits bei Konzentrationen weit unterhalb der Toxizitätsgrenze eine gute Wirkung erzielt werden kann und das einfach durchzuführen ist. Diese Aufgabe wird mit dem Gegenstand der vorliegenden Erfindung gelöst.

Gegenstand der vorliegenden Erfindung ist ein Arzneimittel, enthaltend 1-(N-Butylcarbamoyl)-2-)methoxycarbonylamino)-benzimidazol (Benomyl) und/oder 2-(Methoxycarbonylamino)-benzimidazol (Carbendazim) sowie übliche Hilfs-, Zusatz- oder Trägerstoffe.

Benomyl und Carbendazim sind an sich bekannte Substanzen, sind aber bisher lediglich als fungizide Mittel gegen phytopathogene Pilze in der Landwirtschaft eingesetzt worden (zur Herstellung und Verwendung vergleiche DE-PS 1 956 157, DE-AS 1 932, 297, GB-PS 1 238 977, US-PS 3 637 733, US-PS 3 631 176, US-PS 3 541 213). Carbendazim ist ein wirksamer Metabolit von Benomyl im Organismus der Pflanzen und dort in einer Konzentration von ca. 20 bis 100 ppm wirksam. Anwendungen der genannten Substanzen als Arzneimittel sind bisher nicht bekannt.

Überraschenderweise wurde nun gefunden, daß sich Benomyl und Carbendazim auch hervorragend zur Bekämpfung von Fischkrankheiten, insbesondere zur Bekämpfung von Ichthyophthyriasis eignen. Die bisherigen Versuche mit
den genannten Mitteln deuten darauf hin, daß sie allgemein antiparasitisch, fungizid und bakterizid sind. Die
wirksame Menge (Konzentration im Fischwasser) beträgt
je nach der zu behandelnden Fischart im allgemeinen
0,01 bis 10 ppm, vorzugsweise 0,1 bis 4 ppm. Sie liegt
damit im allgemeinen weit unter der Toxizitätsgrenze
dieser Wirkstoffe, die abhängig von der Fischart, in
der Regel bei über 10 ppm liegt.

Das Mittel eignet sich im wesentlichen zur Anwendung
bei allen Fischarten und anderen Wassertieren, wie
insbesondere allen in der gewerblichen Fischzucht und
Aquakultur und in Heim-, Schauaparien und Teichen gehaltenen Tieren, z. B. Fischen, Invertebraten oder
Lurchen.

Erfindungsgemäß werden die Wirkstoffe als solche in der
entsprechenden Menge dem Hälterungswasser zugegeben.
Die Wirkstoffe können aber auch zusammen mit Futtermitteln oder anderen biologischen Wirkstoffen, wie
Vitaminen zugesetzt werden.

Der Zusatz kann dabei in getrennter Form, d. h. also
jede Substanz einzeln, erfolgen oder aber vorzugsweise
in Form einer vorgefertigten flüssigen oder festen Formulierung, die gegebenenfalls weitere für eine derartige
Formulierung übliche Träger- und/oder Hilfsstoffe enthält. Es kann aber auch der Wirkstoff allein oder in
Form einer solchen Formulierung mit üblichen Träger-
und/oder Hilfsstoffen verwendet werden, wobei infolge
der schlechten Wasserlöslichkeit der Wirkstoffe, insbesondere ein Zusatz eines Dispergier- oder Lösungsmittels zweckmäßig sein kann.

Überraschenderweise wurde gefunden, daß durch einen
Zusatz von Ligninsulfonaten zu den Wirkstoffen besonders
gute Ergebnisse im Hinblick auf die krankheitshemmende
Wirkung erreicht werden, d. h. eine unerwartet hohe
Steigerung der Wirksamkeit, die nicht nur durch die
Wirkung der Ligninsulfonate als Dispergiermittel
erklärbar ist.

Ein weiterer Gegenstand der vorliegenden Anmeldung ist
die Verwendung von 1-(N-Butylcarbamoyl)-2-(methoxycarbonylamino)-benzimidazol und/oder 2-(Methoxycarbonylamino)-benzamidazol als Wirkstoff bei der Bekämpfung
von Krankheiten bei Wassertieren, insbesondere bei der
Bekämpfung der Ichthiophthyriasis, in flüssiger oder
fester Form.

Das erfindungsgemäße Arzneimittel wird vorzugsweise als
flüssiges Wirkstoffkonzentrat in einer Konzentration
von 0,1 bis 80 Gew.-% oder als feste Wirkstoffzubereitung, vorzugsweise in einer Menge von 10 bis 100 Gew.-%,
bezogen auf das gesamte Mittel, in den Handel gebracht.

Als Ligninsulfonate können die üblichen Ligninsulfonate,
wie sie z. B. auch als Dispergiermittel eingesetzt
werden, verwendet werden. Die Menge der Ligninsulfonate
im flüssigen oder festen Mittel beträgt vorzugsweise
0,1 bis 10 und insbesondere 4 bis 8 Gew.-%.

Ein Zusatz von biologisch aktiven Nährstoffen kann
ebenfalls zweckmäßig sein, weil er sich im allgemeinen
positiv auf die Fische auswirkt und den Heilungseffekt
beschleunigt. Unter biologisch aktiven Nährstoffen
werden dabei im allgemeinen Nährstoffe verstanden, die
als Nahrungsergänzungen dienen und die insbesondere eine Zuführung biologisch essentieller und sehr wirksamer Stoffe bewirken. Es ist auch möglich, den Wirkstoff normalen Futtermitteln in einer Konzentration von vorzugsweise 0,1 bis 5 Gew.-% zuzusetzen. Diese werden dann entsprechend dosiert verfüttert.

Neben Nährstoffen kann sich auch ein Gehalt an Mineralien und/oder Vitaminen günstig auswirken.

Das Mittel enthält zweckmäßigerweise auch für derartige Zubereitungen übliche Träger- und/oder Hilfsstoffe. Träger- und Hilfsstoffe sind z. B. solche, wie sie auch für Fungizidformulierungen, wie z. B. für Spritzpulver (feste Zubereitung) oder Emulsionskonzentrate (flüssige Zubereitung) verwendet werden, also z. B. feste Trägerstoffe, Emulgatoren, Emulsionsförderer, Dispergiermittel, Netzmittel, Detergentien, organische Lösungsmittel, Lösungsvermittler usw. oder auch solche Zusätze, wie sie für die Herstellung von Arzneimitteln, insbesondere von Tierarzneimitteln verwendet werden. Die Herstellung der Mittel erfolgt dabei auf an sich übliche Weise, je nach dem Aggregatzustand, z. B. durch Vermischen, Mahlen, Lösen, Emulgieren usw.

Die festen Mittel können als Pulver vorliegen oder aber durch an sich übliche Formgebung, z. B. Tablettierung in eine geeignete Darreichungsform und Dosiereinheit gebracht werden. In flüssiger Form liegt das Mittel vorzugsweise in Form einer Lösung oder eines sogenannten Emulsionskonzentrats vor, wie dies z. B. auch für Fungizide üblich ist. Als Lösungsmittel dienen bevorzugt polare organische Lösungsmittel oder deren Gemische wie z. B. Dimethylsulfoxid oder Alkohol.

Aufgrund der guten Wirksamkeit ist es für den beabsichtigten Erfolg ausreichend, den Wirkstoff oder das
Mittel während weniger Tage einmal am Tag dem Hälterungswasser zuzusetzen; meist reicht ein einziger Zusatz an
einem Tag bereits für die beabsichtigte Wirkung aus.
Die Dosiereinheit wird vorzugsweise so gewählt, daß die
bevorzugt genannten Konzentrationen an Wirkstoff im
Wasser erreicht werden. Menge und Häufigkeit des Zusatzes
sind aber auch abhängig von der Art und Schwere der
Krankheit, von der Art der zu behandelnden Fische, von
der Art des Fischwassers (gewerbliche Fischzuchtgewässer,
Heimaquarien usw.) sowie von der Dauer der Behandlung.

Das erfindungsgemäße Arzneimittel eröffnet aufgrund
seiner hohen Wirksamkeit (Anwendung sehr geringer Konzentrationen an Wirksubstanz weit unterhalb der Toxizitätsgrenze) und der damit verbundenen äußerst
geringen Wasserbelastung einen gut wirksamen und einfachen Weg zur Bekämpfung von Krankheiten. Im Normalfall reicht eine einfache Anwendung aus, wodurch eine
exakte Dosierung möglich ist und eine Kumulierung und
Erreichung toxischer Konzentrationen vermieden werden
kann. Damit lassen sich die aufgezeigten Nachteile
bisher bekannter Bekämpfungsmethoden weitgehend vermeiden.

In den folgenden Beispielen wird die Erfindung und der
damit erreichbare technische Fortschritt näher
beschrieben, ohne die Erfindung darauf zu beschränken.

B e i s p i e l    1

Unter gleichen Bedingungen wurden in drei Fischbehältern
A, B und C (40 Liter) je 16 Sumatrabarben ausgesetzt.
Die Wassertemperatur betrug 25 $\pm$ 1°C. Nach eintägiger
Haltung wurden die Fische in Kontakt mit Ichthiophthyriasis auslösenden Parasiten gebracht. Nach visuell
sichtbarer Infektion wurden zugesetzt:

Im Aquarium A: Reines Wasser

Im Aquarium B: 1-(N-Butylcarbamoyl)-2-(methoxycarbonylamino)-benzimidazol (als pulverförmiges
Gemisch. Konzentration an Wirkstoff 0,2
mg/ml)

Im Aquarium C: Malachitgrün (Wirkstoffkonzentrat 0,03
mg/ml)

Die Zugabe wurde mit der halben Dosierung nach 3 bis 5
Tagen wiederholt.

6 Tage nach Behandlungsbeginn waren im Aquarium A alle
Tiere tot.

Im Aquarium B und C hatte sich das Schuppenbild normalisiert. Die Ausfälle lagen bei je 2 Tieren pro Becken.

Ein Vergleich dieser Ergebnisse zeigt deutlich die gute
Wirkung des erfindungsgemäßen Mittels.

Beispiel    2

Unter den Bedingungen des Beispiels 1 wurden jeweils 18 Neonsalmler behandelt. Die Nachdosierung erfolgte jedoch in Höhe der Anfangsdosierung.

Ergebnis nach 7 Behandlungstagen:
Aquarium A: Totalausfall
Aquarium B: Keine Ausfälle
Aquarium C: Ein Ausfall.

P a t e n t a n s p r ü c h e

1. Arzneimittel, enthaltend 1-(N-Butylcarbamoyl)-2-
   (methoxycarbonylamino)-benzimidazol und/oder
   2-(Methoxycarbonylamino)-benzimidazol sowie
   übliche Hilfs-, Zusatz- oder Trägerstoffe.

2. Arzneimittel nach Anspruch 1, enthaltend zusätzlich Ligninsulfonat.

3. Arzneimittel nach Anspruch 1 oder 2 in wäßriger
   Suspension mit einer Wirkstoffkonzentration von
   0,1 bis 20 mg/ml oder gegebenenfalls mit Lösungsvermittler bis 20 mg/ml.

4. Arzneimittel nach Anspruch 1,    d a d u r c h
   g e k e n n z e i c h n e t ,    daß es in einem
   polaren Lösungsmittel in praktisch konzentrierter
   Form oder in Mischung mit Feststoffen vorliegt.

5. Arzneimittel nach Anspruch 1,    d a d u r c h
   g e k e n n z e i c h n e t ,    daß es einem
   Futtermittel beigemischt ist.

6. Verwendung von 1-(N-Butylcarbamoyl)-2-(methoxycarbonylamino)-benzimidazol und/oder 2-(Methoxycarbonylamino)-benzimidazol bei der Bekämpfung von
   Erkrankungen von Wassertieren.

7. Verwendung von 1-(N-Butylcarbamoyl)-2-(methoxycarbonylamino)-benzimidazol und/oder 2-Methoxy-
   carbonylamino)-benzimidazol nach Anspruch 6 bei
   der Bekämpfung von Ichthyophthyriasis bei Fischen.

**Europäisches Patentamt**

**EUROPÄISCHER RECHERCHENBERICHT**

**0167710**
Nummer der Anmeldung

EP 85 10 3325

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 059 074 (SANKYO CO. LTD.) * Seiten 13-15, 17-21 * | 1 | A 61 K 31/415// C 07 D 235/32 |
| A | DE-B-2 909 550 (BAYER) | | |
| A | CHEMICAL ABSTRACTS, Band 95, Nr. 1, 6. Juli 1981, Seite 130, Nr. 1114p, Columbus, Ohio, US; J.V. BELL u.a.: "Some microbial contaminants and control agents in a diet and larvae of Heliothis spp." & J. INVERTEBR. PATHOL. 1981, 37(3), 243-8 | | |
| A | CHEMICAL ABSTRACTS, Band 89, Nr. 21, 20. November 1978, Seite 124, Nr. 174337t, Columbus, Ohio, US; L. MIR u.a.: "Action of antimicrotubular drugs on Physarum polycephalum" & MICROBIOS LETT. 1977, 5(17), 39-44 | | RECHERCHIERTE SACHGEBIETE (Int. Cl.4) C 07 D 235/00 A 61 K 31/00 |

Der vorliegende Recherchenbericht wurde für alle Patentansprüche erstellt.

| Recherchenort DEN HAAG | Abschlußdatum der Recherche 26-06-1985 | Prüfer DE BUYSER I.A.F. |
|---|---|---|